(19) Europäisches Patentamt
European Patent Office
Office européen des brevets

(11) Publication number: **0 116 401**
A2

# (12) EUROPEAN PATENT APPLICATION

(21) Application number: 84300107.4

(22) Date of filing: 09.01.84

(51) Int. Cl.³: A 61 K 47/00
A 61 K 9/08

(30) Priority: 10.01.83 GB 8300529
14.06.83 GB 8316204

(43) Date of publication of application:
22.08.84 Bulletin 84/34

(84) Designated Contracting States:
DE FR IT

(71) Applicant: ROBERT YOUNG & COMPANY LIMITED
Cranstonhill Chemical Works Elliot Street
Glasgow G3 8JT Scotland(GB)

(72) Inventor: Ballany, John McKeller
12c Darroch Way
Cumberbauld Scotland(GB)

(72) Inventor: Henderson, David Jenkins
96 Longholme Road
Carlisle(GB)

(74) Representative: King, James Bertram
Herbert J.W. Wildbore Wardrobe Court 146a Queen
Victoria Street
London, EC4V 5AT(GB)

## (54) Endoparasiticidal compositions.

(57) Formulation for treating helminthic parasites by application of levamisole to the skin or fleece of the animal by a localised pour-on method. The formulation comprising 2-20% levamisole in a solvent comprising a monoterpene fraction, particularly terpinolene, or a solvent comprising a mixture of alkylbenzenes higher than xylene, such as Shellsol AB. The solvent may be diluted with a further bland or carrier solvent. The solvent being effective to transmit the levamisole through the skin of the animal without substantial harmful effects.

EP 0 116 401 A2

"Endoparasiticidal Compositions"

This invention relates to endoparasiticidal compositions, particularly to compositions for combating helminthic parasites by application of the composition to a localised area of an animal by a so-called "pour on" or "spot on" method. In such a method the active compound dissolved or suspended in a suitable solvent or carrier is poured directly on to the skin or fleece of an infested animal, for example along the back line, or as a discrete locally applied spot with the active compound being systemically absorbed by transmission through the animal's skin.

The effectiveness of the compound "levamisole" when applied to the skin of an animal is known, but serious problems occur with pour on methods due to the intense localised application which frequently results in severe skin damage or high irritation levels brought about the solvent/active combination.

To combat helminthic parasites in cattle we have proposed levamisole in butyl dioxitol as solvent, this compound proving to produce little or no irritancy on the skin of cattle whilst also producing effective transport of the active levamisole compound through the cattle skin. With sheep, however, the aforesaid product has proved to be ineffective and we believe this to be due to the fact that the transporting effect of this levamisole plus solvent combination through the cattle skin barrier is not operative on sheep. Many solvent compounds taught by the prior art as

being useful have been tested and found to produce inadequate worming results, and in many instances considerable skin damage has been shown to exist.

In selecting and assessing suitable solvents not only does the question of efficiency in transport through the skin barrier and lack of damage or irritation to the animal have to be considered, but also the stability of the formulation under practical storage conditions encountered in the field has to be taken into account.

It is therefore an object of this invention to provide a novel composition containing levamisole which is effective in particular on sheep for combating endoparasites.

A further object of the invention is to provide a method of systemically combating helminthic parasites in non-human animals, especially sheep using a levamisole containing formulation.

According to one aspect of this invention there is provided a formulation for application to the skin of an animal to combat helminthic parasites systemically, the composition comprising a systemically effective proportion of levamisole in a solvent comprising a monoterpene fraction. In a second preferred aspect the solvent comprises an aromatic hydrocarbon distillation cut having a boiling point range between about 186 and 205°C and containing as major constituent benzene rings substituted with $C_3$ and $C_4$ side chains and being effective to transmit the levamisole through the skin of an animal to which the formulation is applied.

Preferably the monoterpene fraction is substituted by the said aromatic hydrocarbon cut either wholly or in part. The terpene fraction may comprise an eucalyptus oil. The effectiveness of formulations above mentioned was most surprising as from a very large number of solvents tried very few proved to produce any effective results against worms except for xylene, which latter compound, however, produced totally unacceptable skin damage. It was therefore most surprising that the above compounds were not only effective in producing good worm results, but also could be formulated to avoid significant skin or other damaging effect on sheep.

The solvents according to the invention could more broadly be described and defined in the second aspect as a mixture of higher alkylbenzenes than xylene, and in the first aspect as a monoterpene fraction. The definition of such solvents is to be interpreted in a broad sense as describing mixtures which are effective and in accordance with the surprising discovery of the present invention. Preferably the second aspect solvent will comprise a solvent known commercially as "Shellsol AB", the first aspect solvents may be commercially available eucalyptus oil. As an alternative to Levamisole we propose also tetramisole.

In a preferred formulation according to the invention the amount of solvent is minimised by concentrating the levamisole content as far as possible to minimise the solvent does and by diluting the solvent with a co-solvent or carrier such as butyl dioxitol. The levamisole content

may be between 2 and 20%, preferably 5-12%, using Shellsol AB to make up the balance. A further formulation may comprise a levamisole content of 2-20%, preferably 5-12%, with a eucalyptus oil content of 10-40% and up to 2% phenylbutazone with the balance being butyl dioxitol or other bland solvent. The formulation may further include a component comprising a pyrethroid such as cypermethrin, or an avermectin such as avermectin B1. In the former case, stablisation of the formulation by inclusion of an acid would be required, see GB 8219104 the text of which is appended for supporting such a combination.

Two examples of formulations according to the invention are as follows:

1) Levamisole 5 - 12%
   Shellsol AB balance

2) Levamisole 5%
   Eucalyptus oil 20%
   Phenylbutazone 1%
   Butyldioxitol balance

Both products reduced egg counts of worm by 90% in sheep with clinically severe infestations.

The first was safe up to 2 x dose rate, although it had a mild skin irritation. The second had enough eucalyptus oil to eliminate worms, and the presence of phenylbutazone, a recognised anti-irritant, reduced the tendency for skin irritation.

Workable formulae will comprise generally 1) a levamisole content of 2-20%, preferred 5-12%, with Shellsol AB making up

the balance, and 2) a levamisole content of 2-20%, preferred 5-12%, a eucalyptus oil content of 10-40%, a phenylbutazone content up to 2%, with the balance in butyl dioxitol or other bland solvent. Also any mixture of the two principal solvents is feasible provided it permits effective skin penetration by levamisole, and any fraction (or chemical entity) chemically similar to either Shellsol AB or eucalyptus oil may be utilisable.

A pyrethroid presence (such as cypermethrin) with suitable stabilisation by acid incorporation, and/or avermectin, may be included in the above.

The latter mentioned formulations are particularly useful in eliminating both endo- and ecto-parasites from treated animals.

The two preferred solvents referred to herein are more specifically defined by the following:

Eucalyptus Oil

Consists mainly of cineoles, and α pinene

Cineoles have the formulae:-

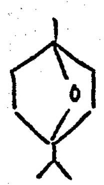 and 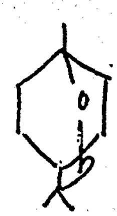 an ether

whereas α - pinene is 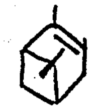 a hydrocarbon

On this basis all pine oils, lemon oils, citronellas etc., are sources of such monoterpenes, may prove suitable in this application either singly or in combination. Accordingly the use of monoterpene fractions, as

instanced by eucalyptus oil and various essential oils is included.

This covers structural types and functional groups. Structures are:- 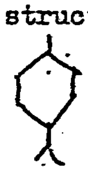 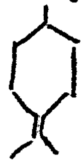 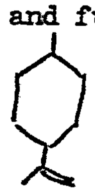 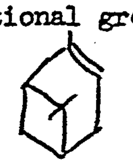 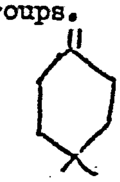

with ethers, alcohols, ketones, hydrocarbons & aldehydes occurring, with usually at least two functional groups present in any commercial oil.

<u>Shellsol AB</u>

The above is a distillation cut boiling over the range 186-205°C containing ≥ 99% aromatic hydrocarbons, the major constituents being benzene rings substituted with $C_3$ and $C_4$ side chains.

e.g. $C_9$ fraction 35%

constituents n-propylbenzene 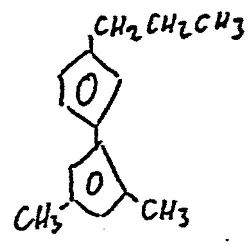

trimethylbenzene isomers e.g.

indane 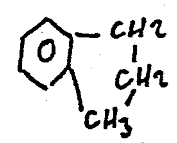

$C_{10}$ fraction 49%

constituents ethyldimethylbenzene isomers e.g. 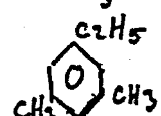

tetramethylbenzene isomers 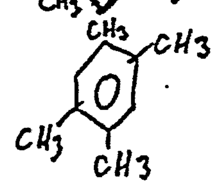

$C_{11}$ fraction 9%

constituents diethyl methyl benzene isomers 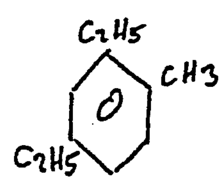

There is also a minor proportion (7%) of naphthalene and methylnaphthalene

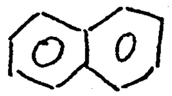

and

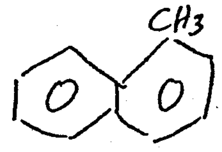

Example 1

Method:

Four lambs were faecal sampled. Their e.p.g. outputs were evaluated by a modified McMaster technique. The lambs were treated with 5% levamisole Shellsol AB, and the formulations were applied as a single application of 10 mg/kg at skin level, between the scapulae. E.p.g. outputs were assessed 7 and 13 days post-treatment.

| Animal No. | Body wt. (Kg.) | Material | Quantity applied (ml) |
|---|---|---|---|
| 1 | 38 | PA/S/16 | 7.6 |
| 2 | 36 | " | 7.2 |
| 3 | 32 | " | 6.4 |
| 4 | 27 | " | 5.4 |

Results: E.p.g. counts.

| | Pre-treatment | | 7 days Post-treatment | |
|---|---|---|---|---|
| Animal No. | Trich. | Nematodirus | Trich. | Nematodirus |
| 1 | 1100 | 0 | 0 | 0 |
| 2 | 1000 | 150 | 0 | 0 |
| 3 | 800 | 0 | 0 | 0 |
| 4 | 1650 | 0 | 0 | 0 |

13 days post-treatment

| Animal Number | Trich. | Nematodirus |
|---|---|---|
| 1 | 0 | 0 |
| 2 | 0 | 0 |
| 3 | 0 | 0 |
| 4 | 50 | 0 |

Conclusions : % change in e.p.g. post-treatment.

Comments: The formulation produced a slight skin reaction that was considered acceptable.

The skin reactions were observed over the next 4 - 5 weeks for any further damage to the skin or wool. None was observed.

Example 2

The formulation used was 5% Levamisole base, 20% Eucalyptus oil, remainder Butyl dioxitol.

Method:

Lambs were faecal sampled and e.p.g. found as indicated below. The animals were treated along the mid backline.

| Animal No. | Body Wt. (kg) | Product Vol.(ml) | Pre-treatment E.P.G. | Post-treatment E.P.G. |
|---|---|---|---|---|
| 1 | 31 | 6.2 | 900 | 0 |
| 2 | 15 | 3.0 | 1650 | 0 |
| 3 | 22 | 4.4 | 1250 | 0 |
| | | | 1267 | 0 |

Conclusions:

The formulation produced 100% reduction in heavily infested lambs. The assessment of skin reactions was favourable.

Example 3

Formulation: 12% Levamisole base, remainder Shellsol AB.

Object: to asses the efficacy at 5, 7·5 and 10 mg/kg

Method:

17 sheep were faecal sampled. Their e.p.g. outputs were assessed by a modified McMaster technique. The appropriate volume of formulation was applied as a spot treatment at skin level, over the rump. Worm egg outputs were re-assessed 6 and 14 days post-treatment.

| Animal No. | Body Wt.(kg) | Application rate | Quantity applied (ml) |
|---|---|---|---|
| 1 | 40 | 5mg/kg | 1.7 |
| 2 | 30 | " | 1.25 |
| 3 | 43 | " | 1.8 |
| 4 | 40 | " | 1.7 |
| 5 | 30 | " | 1.25 |
| 6 | 30 | 7.5 mg/kg | 1.7 |
| 7 | 35 | " | 2.0 |
| 8 | 31 | " | 1.6 |
| 9 | 32 | " | 1.8 |
| 10 | 37 | " | 2.0 |

| Animal No. | Body Wt.(kg) | Application rate | Quantity applied (ml) |
|---|---|---|---|
| 11 | 34 | 10 mg/kg | 2.8 |
| 12 | 41 | " | 3.5 |
| 13 | 30 | " | 2.5 |
| 14 | 35 | " | 3.0 |
| 15 | 41 | " | 3.5 |
| 16 | - | Controls | |
| 17 | - | " | |

Results: e.p.g. counts:

| Animal No. | Trichostrongyl e.p.g. Pre-treatment | 6 days | 14 days post-treatment |
|---|---|---|---|
| 1 | 2800 | 200 | 50 |
| 2 | 1150 | 150 | 0 |
| 3 | 950 | 50 | 0 |
| 4 | 650 | 0 | 0 |
| 5 | 400 | 250 | 400 |
| Mean | 1190 | 130 | 90 |
| 6 | 1850 | 100 | 150 |
| 7 | 950 | 50 | 100 |
| 8 | 850 | 50 | 0 |
| 9 | 600 | 50 | 50 |
| 10 | 300 | 0 | 0 |
| Mean | 910 | 50 | 60 |

| Animal No. | Pre-treatment | 6 days | 14 days post-treatment |
|---|---|---|---|
| 11 | 1150 | 0 | 0 |
| 12 | 950 | 0 | 0 |
| 13 | 700 | 100 | 0 |
| 14 | 500 | 0 | 0 |
| 15 | 300 | 0 | 0 |
| Mean | 720 | 20 | 0 |
| 16 | 800 | 1400 | 1800 |
| 17 | 750 | 800 | 450 |
| Mean | 775 | 1100 | 1125 |

Conclusions: Table of % reductions:

| Application rate | 6 days post-treatment | 14 days post-treatment |
|---|---|---|
| 5mg/kg | 89 | 92 |
| 7.5 mg/kg | 94 | 93 |
| 10 kg/kg | 97 | 100 |

There was an increase in the control Mean during the trial period.

Examples 4, 5 and 6

Further examples of formulation are defined in the following table:

| | Ex. 4 | Ex. 5 | Ex. 6 |
|---|---|---|---|
| levamisole | 8 | 8 | 8 |
| eucalyptus oil | 39 | - | - |
| "terpinolene" | - | 71 | 73 |
| dimethylsulphoxide | 30 | - | 10 |
| butyl dioxitol | 20 | - | - |
| shellsol AB | - | 10 | - |
| | optional further additive | | |
| butylated hydroxy toluene | 1 | 1 | 1 |
| phenylbutazone (as anti-irritant) | 1 | 1 | 1 |

All quantities are in gm per 100 ml.

"Terpinolene" is a trade name given to a by-product of terpineol synthesis containing the hydrocarbons, limonene and terpinolene, ethers such as the cineoles, and the alcohol terpineol, as in the commercial grade of terpinolene sold by Zimmerman-Hobbs Limited.

In the above formulations, all are stable, efficaceous, and relatively free from skin irritation effects, the presence of BHT (butylated hydroxy toluene) avoids possible peroxide build-up with

subsequent oxidation of levamisole, and the phenylbutazone, used to counter dermal irritation, is included to reduce still further the potential to irritate skin.

Preferably a "terpinolene" product having a composition substantially as mentioned herein or as commercially available is used as solvent and being a further development of the terpene fractions previously mentioned.

CLAIMS

1. A formulation for application to the skin of an animal to combat helminthic parasites systemically, the composition comprising a systemically effective proportion of levamisole in a solvent comprising a monoterpene fraction.and being effective to transmit the levamisole through the skin of an animal to which the formulation is applied.

2. A formulation in accordance with claim 1, wherein the terpene fraction comprises a eucalyptus oil.

3. A formulation in accordance with claims 1 or 2, comprising:-

levamisole 2 - 20%

eucalyptus oil 10 - 40%

and a balance comprising a bland solvent or carrier.

4. A formulation according to claim 3, comprising:-

levamisole 5 - 12%

eucalyptus oil 20%

5. A formulation according to claim 3 or 4, wherein the bland solvent comprises butyl dioxitol.

6. A formulation according to any preceding claim, containing up to 2% phenylbutazone as anti-irritant, and/or up to 2% butylated hydroxy toluene as anti-oxidant.

7. A formulation according to any preceding claim, wherein the monoterpene fractions comprise primarily cineoles and $\alpha$ pinene.

8. A formulation according to any preceding claim, wherein the terpene fraction comprises a terpinolene product.

9. A formulation for application to the skin of an animal to combat helminthic parasites systemically, the formulation comprising a systemically effective proportion of levamisole in a solvent comprising an aromatic hydrocarbon distillation cut having a boiling point range between about 186 and 205$^{o}$C and containing as major constituent benzene rings substituted with $C_3$ and $C_4$ side chains and being effective to transmit the levamisole through the skin of an animal to which the formulation is applied.

10. A formulation in accordance with claim 9, wherein the solvent comprises a mixture of alkylbenzenes higher than xylene.

11. A formulation in accordance with claim 10, wherein the solvent is Shellsol AB.

12. A formulation in accordance with claims 9 or 10 or 11 wherein the composition comprises:-

levamisole 2 - 20%

with the balance comprising Shellsol AB.

13. A formulation according to any one of claims 1 to 8, wherein the terpene fraction is replaced in part by the solvent of any one of claims 8 to 11.

14. A formulation according to any preceding claim, wherein the levamisole is replaced, wholly or in part, by tetramisole.

15. A formulation in accordance with any preceding claim, wherein a pyrethroid is included.

16. A formulation according to claim 15, wherein the pyrethroid is stabilised by an acid.

17. A formulation according to any preceding claim, wherein avermectin is included.

18. A method of systemically combating helminthic parasites in non-human animals, especially sheep using a levamisole containing formulation, the formulation being as claimed in any one of the preceding claims, the method comprising applying the formulation directly to the skin or fleece of the animal.

19. A method in accordance with claim 18, wherein the formulation is applied along the back-line or at a discrete spot on the animal.

20. A method in accordance with claims 18 or 19, wherein the animal is a sheep.

21. A method in accordance with any preceding claim, wherein the formulation is applied to the animal at a rate between 3 and 15 mg/Kg of body weight.